Europäisches Patentamt

European Patent Office

Office européen des brevets

(11) Publication number: **0 087 744**
**B1**

# EUROPEAN PATENT SPECIFICATION

(45) Date of publication of patent specification: **26.11.86**

(21) Application number: **83101742.1**

(22) Date of filing: **23.02.83**

(51) Int. Cl.⁴: **A 61 K 35/78,** A 61 K 31/70,
A 61 K 31/52 // (A61K35/78,
31:70, 31:52)

(54) **Lipid metabolism improving agents.**

(30) Priority: **03.03.82 JP 34526/82**

(43) Date of publication of application:
**07.09.83 Bulletin 83/36**

(45) Publication of the grant of the patent:
**26.11.86 Bulletin 86/48**

(84) Designated Contracting States:
**AT CH DE LI**

(56) References cited:
**ROTE LISTE, 1963, Edit. Cantor,
Aulendorf/Württ.,page 61: "Apoplectal-
Kapseln"**

**ROTE LISTE, 1963, Edit. Cantor,
Aulendorf/Württ., page 61: "Apoplectal-
Ampullen"**

(73) Proprietor: **NIPPON SHINYAKU COMPANY,
LIMITED
14, Kisshoin Nishinosho Monguchicho
Minami-ku Kyoto-shi Kyoto (JP)**
(73) Proprietor: **Klinge Pharma GmbH
Berg-am-Laim-Strasse 129
D-8000 München 80 (DE)**

(72) Inventor: **Kumagai, Akira
16-3-1002 Masago-3-chome
Chiba (JP)**
Inventor: **Saito, Yasushi
4-22 Katsuragi-2-chome
Chiba (JP)**

(74) Representative: **Wey, Hans-Heinrich, Dipl.-Ing.
et al
Patentanwälte Müller-Börner & Wey
Widenmayerstrasse 49
D-8000 München 22 (DE)**

Courier Press, Leamington Spa, England.

**0 087 744**

**Description**

The present invention relates to the use of horse-chestnut seed extract, oxyethophylline and adenosine for the manufacture of a composition for increasing high density lipoprotein cholesterol in blood. The invention is particularly directed to lipid metabolism improving agents which increase high density lipoprotein (hereinafter abbreviated as HDL) cholesterol in blood.

For a long time attention has been focussed on abnormal lipid metabolism as a general cause of modern diseases. Normalization of the lipid metabolism abnormality is one of the most important means attempted in clinical fields in order to remove or alleviate fatal factors in various diseases.

Recently it has been reported that there is a negative interrelation between HDL cholesterol and ischemic heart diseases and, since then, attempts have been made in therapy and prevention of various diseases using HDL cholesterol as a target.

On the other hand, pharmaceutical preparations containing horse-chestnut seed extract, oxyethophylline and adenosine are known as "Apoplectal®" and have been used as remedies for apoplexy and brain circulation disorders. However, there is no pharmacological relation between the known action (a dilation of blood vessels) and an increase in HDL cholesterol in blood.

The present inventors have conducted extensive studies keeping the above importance of HDL cholesterol in clinical fields in mind and, as a result, found that pharmaceutical preparations containing horse chestnut (Roßkastanie) extract, oxyethophylline and adenosine greatly increase HDL cholesterol.

Clinical effects of the present invention are as follows.

(1) Methods

Patients who satisfy either the condition that their total cholesterol concentration is 220 mg/dl or more or the condition that their neutral fat concentration is 140 mg/dl or more are selected from out-patients and they are given a pharmaceutical preparation containing horse chestnut seed extract, oxyethophylline and adenosine in 15:55:1.5 ratio by weight. Ages of such selected out-patients are from 26 to 69 years old. They consist of twelve males and eight females. Duration of the administration is sixteen weeks. Blood samples are collected every four weeks and concentrations of total cholesterol, neutral fat and HDL cholesterol in the collected blood are determined.

Methods for the determination are as follows. Determination of cholesterol and neutral fat is carried out by an enzyme method; determination of HDL cholesterol is done by a heparin-manganese method. The dose of the pharmaceutical preparation is six capsules per day (each capsule contains 71.5 milligrams of the active ingredients) being given three times a day after each meal.

(2) Results

In Table 1 are given ages, sex and changes in blood lipid levels.

1. Effect of the pharmaceutical composition on total cholesterol. (Tables 2 and 3)

When all cases tested are taken into consideration, the initial concentration was 211 ± 45 mg/dl and, after sixteen weeks, it was lowered to 201 ± 36 mg/dl and, therefore, the decrease is 4.7% from the initial concentration.

When the so-called hypercholesterolemia cases, where the initial concentration is not lower than 220 mg/dl, are taken into consideration, the numbers of the cases was ten and their initial concentration is 246 ± 26 mg/dl. The concentrations after four, eight, twelve and sixteen weeks are 230 ± 24, 226 ± 15, 232 ± 16 and 229 ± 18 mg/dl, respectively. Thus, after eight and twelve weeks, there are significant lowering ($P < 0.05$); after four and sixteen weeks, there are lowering tendencies. The decrease in the sixteenth week compared with the initial concentration is 6.9%. It is characteristic that the decrease is observed from an early stage of the fourth week and continues thereafter.

2. Effect of the pharmaceutical composition on neutral fat (Tables 4 and 5).

When all cases tested are taken into consideration, the initial concentration was 174 ± 76 mg/dl and after four, eight, twelve, and sixteen weeks, they were 179 ± 89, 172 ± 84, 176 ± 82 and 168 ± 69 mg/dl, respectively. Thus significant decreases were observed after eight weeks ($P < 0.01$) and sixteen weeks ($P < 0.05$).

When the hypercholesterolemia cases, where the initial concentration is not lower than 140 mg/dl, are taken into consideration, the initial concentration is 217 ± 54 mg/dl and the concentrations after four, eight, twelve and sixteen weeks were 227 ± 69, 207 ± 84, 210 ± 80 and 201 ± 63 mg/dl, respectively. Thus, a significant decreases ($P < 0.01$) was observed after eight weeks and, after sixteen weeks, the decrease from the initial concentration is about 7.4%.

3. Effect of the pharmaceutical composition on HDL cholesterol (Tables 6 and 7).

When all cases tested are taken into consideration, the initial concentration was 40 ± 6 mg/dl and, after four, eight, twelve and sixteen weeks, the concentrations were 44 ± 8, 44 ± 6, 45 ± 7 and 46 ± 7 mg/dl and all show a significant increase (four weeks, $P < 0.05$; eight weeks and sixteen weeks, $P < 0.001$; twelve weeks, $P < 0.01$).

When the low HDL cholesterol level group, where the initial concentration is not higher than 40 mg/dl, is taken into consideration, the initial concentration was 36 ± 4 mg/dl and, after four, eight, twelve and sixteen weeks, the concentrations were 41 ± 9, 41 ± 5, 42 ± 6 and 43 ± 5 mg/dl. Thus, after four weeks, an

2

**0 087 744**

increasing tendency was observed and then a significant increase now seen (eight weeks, $P < 0.01$; twelve weeks, $P < 0.05$; sixteen weeks, $P < 0.01$).

As obvious from the above-given clinical results, the increase in HDL cholesterol concentration caused by present pharmaceutical composition is remarkable. In cases where HDL cholesterol is low, the effect is especially strong and the utility is much more than would be expected when compared with the conventional lipid metabolism improving agents known in the art.

Such an increasing action is probably due to lowering action for TG or to metabolism acceleration of VLDL and, in addition, due to synthesis of apoproteins caused by, for example, adenosine.

The mixing ratio of horse chestnut seed extract, oxyethophylline and adenosine used in the present invention may optionally be set and the ratio of 15:55:1.5 by weight is particularly preferred. Owing to its characteristic properties, the present pharmaceutical composition is preferably administered as capsules. It is preferred that the dose of the present pharmaceutical composition is about six capsules a day (each capsule contains 71.5 mg of active ingredients) when the mixing ratio is as given above. However, the dose may be increased or decreased depending upon the symptoms.

The present pharmaceutical composition is available as "Apoplectal" (Registered Trade Mark) as a remedy for apoplexy and circulation disorder and is widely used. Therefore it is quite easily obtainable.

Acute toxicities of the present pharmaceutical composition are as given in Table 8 and are quite safe. When this pharmaceutical composition is administered continuously per os to rats (both male and female) at 50, 200 and 500 mg/kg for ninety days, no abnormality is observed in body weights, amounts of food and water consumed, weights of organs, blood, and pathoanatomic observations except that, in a group administered with 500 kg/mg, an increase in urine is noted.

3

TABLE 1

| Case Number | Age | Sex | Total Cholesterol | | | | | Neutral Fat | | | | | HDL Cholesterol | | | | |
|---|---|---|---|---|---|---|---|---|---|---|---|---|---|---|---|---|---|
| | | | Initial Concn | 4 | 8 | 12 | 16 (weeks) | Initial Concentration | 4 | 8 | 12 | 16 (weeks) | Initial Concentration | 4 | 8 | 12 | 16 (weeks) |
| No. 1 | 45 | M | 196 | 200 | 208 | 204 | 206 | 196 | 200 | 208 | 204 | 206 | 38 | 38 | 40 | 39 | 40 |
| 2 | 43 | M | 214 | 177 | 188 | 182 | 181 | 214 | 177 | 188 | 182 | 181 | 34 | 34 | 38 | 40 | 42 |
| 3 | 40 | M | 192 | 178 | 180 | 195 | 188 | 192 | 178 | 180 | 195 | 188 | 42 | 40 | 46 | 54 | 50 |
| 4 | 40 | M | 178 | 166 | 164 | 158 | 161 | 178 | 166 | 164 | 158 | 161 | 34 | 44 | 42 | 46 | 50 |
| 5 | 45 | M | 158 | 196 | 182 | 189 | 186 | 158 | 196 | 182 | 189 | 186 | 38 | 64 | 48 | 56 | 52 |
| 6 | 42 | M | 159 | 177 | 164 | 166 | 168 | 159 | 177 | 164 | 166 | 168 | 34 | 48 | 46 | 45 | 44 |
| 7 | 69 | M | 243 | 232 | 221 | 226 | 224 | 243 | 232 | 221 | 226 | 224 | 40 | 40 | 42 | 40 | 44 |
| 8 | 56 | F | 223 | 216 | 209 | 227 | 218 | 223 | 216 | 209 | 227 | 218 | 46 | 50 | 50 | 50 | 50 |
| 9 | 32 | F | 182 | 170 | 176 | 173 | 174 | 182 | 170 | 176 | 173 | 174 | 42 | 46 | 44 | 44 | 44 |
| 10 | 65 | F | 226 | 202 | 214 | 221 | 193 | 249 | 202 | 214 | 221 | 193 | 38 | 32 | 38 | 40 | 38 |
| 11 | 45 | F | 286 | 269 | 251 | 264 | 258 | 286 | 269 | 251 | 264 | 258 | 28 | 30 | 32 | 34 | 38 |
| 12 | 26 | M | 141 | 145 | 132 | 132 | 142 | 233 | 202 | 218 | 210 | 218 | 41 | 44 | 43 | 45 | 48 |
| 13 | 43 | M | 246 | 210 | 221 | 233 | 245 | 141 | 145 | 132 | 132 | 142 | 39 | 40 | 38 | 32 | 42 |
| 14 | 46 | M | 132 | 124 | 126 | 130 | 128 | 246 | 210 | 221 | 233 | 245 | 44 | 46 | 50 | 54 | 66 |
| 15 | 33 | M | 223 | 240 | 232 | 236 | 234 | 132 | 124 | 126 | 130 | 128 | 40 | 40 | 43 | 46 | 44 |
| 16 | 67 | M | 279 | 255 | 230 | 244 | 237 | 223 | 240 | 232 | 236 | 234 | 48 | 44 | 46 | 45 | 45 |
| 17 | 61 | F | 220 | 218 | 220 | 219 | 220 | 182 | 156 | 129 | 143 | 136 | 46 | 55 | 51 | 53 | 52 |
| 18 | 58 | F | 223 | 224 | 222 | 223 | 223 | 98 | 81 | 90 | 94 | 88 | 32 | 42 | 37 | 40 | 38 |
| 19 | 62 | F | 178 | 183 | 169 | 176 | 173 | 189 | 105 | 98 | 96 | 100 | 53 | 57 | 55 | 54 | 55 |
| 20 | 43 | F | 268 | 263 | 258 | 253 | 248 | 171 | 218 | 152 | 185 | 169 | 48 | 48 | 44 | 46 | 46 |

**0 087 744**

### TABLE 2
Total Cholesterol (mg/dl) (all groups) (n = 20)

|  | Initial Concentration | Four Weeks | Eight Weeks | Twelve Weeks | Sixteen Weeks | thereafter |
|---|---|---|---|---|---|---|
| Average | 211 ± 45 | 202 ± 38 | 199 ± 36 | 203 ± 38 | 201 ± 36 | |
| P | — | 0.1154 | 0.0112* | 0.0661 | 0.0566 | |

(*: P < 0.05)

### TABLE 3
Total Cholesterol (mg/dl) (hypercholesterolemia group with not less than 220 mg/dl) (n = 10)

|  | Initial Concentration | Four Weeks | Eight Weeks | Twelve Weeks | Sixteen Weeks | thereafter |
|---|---|---|---|---|---|---|
| Average | 246 ± 26 | 230 ± 24 | 226 ± 15 | 232 ± 16 | 229 ± 18 | |
| P | — | 0.0673 | 0.0140* | 0.0462* | 0.0527 | |

(*: P < 0.05)

### TABLE 4
Neutral Fat (mg/dl) (all groups) (n = 20)

|  | Initial Concentration | Four Weeks | Eight Weeks | Twelve Weeks | Sixteen Weeks | thereafter |
|---|---|---|---|---|---|---|
| Average | 174 ± 76 | 179 ± 89 | 172 ± 84 | 176 ± 82 | 168 ± 69 | |
| P | — | 0.0963 | 0.0064** | 0.0612 | 0.0304* | |

(*: P < 0.05) (**: P < 0.01)

### TABLE 5
Neutral Fat (mg/dl) (group of cases with high neutral fat blood symptoms with not less than 140 mg/dl) (n = 18)

|  | Initial Concentration | Four Weeks | Eight Weeks | Twelve Weeks | Sixteen Weeks | thereafter |
|---|---|---|---|---|---|---|
| Average | 217 ± 54 | 227 ± 69 | 207 ± 84 | 210 ± 80 | 201 ± 63 | |
| P | — | 0.1371 | 0.0088** | 0.0692 | 0.2742 | |

(**: P < 0.01)

### TABLE 6
HDL Cholesterol (mg/dl) (all groups) (n = 20)

|  | Initial Concentration | Four Weeks | Eight Weeks | Twelve Weeks | Sixteen Weeks | thereafter |
|---|---|---|---|---|---|---|
| Average | 40 ± 6 | 44 ± 8 | 45 ± 7 | 46 ± 7 | | |
| P | — | 0.0268* | 0.0008*** | 0.0012** | 0.0003*** | |

(*: P < 0.05)
(**: P < 0.01)
(***: P < 0.001)

5

TABLE 7
HDL Cholesterol (mg/dl) (group of cases with low HDL cholesterol
concentrations of not more than 40 mg/dl) (n = 11)

|  | Initial Concentration | Four Weeks | Eight Weeks | Twelve Weeks | Sixteen Weeks | thereafter |
|---|---|---|---|---|---|---|
| Average | 36 ± 4 | 41 ± 9 | 41 ± 5 | 42 ± 6 | 43 ± 5 | |
| P | — | 0.0866 | 0.0045** | 0.0182* | 0.0010** | |

(*: P < 0.05)
(**: P < 0.01)

TABLE 8
Acute Toxicities of the Present Invention Pharmaceutical (LD$_{50}$ — mg/kg)
(Horse Chestnut Seed Extract:Oxyethophylline:Adenosine = 15:55:1.5)

|  | Administration Routes | per os | intraperitoneal |
|---|---|---|---|
| Animals (Mice) | ♂ | 1437 | 411 |
|  | ♀ | 1485 | 391 |

**Claims**

1. The use of horse-chestnut seed extract, oxyethophylline and adenosine for the manufacture of a composition for increasing high density lipoprotein cholesterol in blood.
2. The use according to·claim 1 in which the horse chestnut seed extract, oxyethophylline and adenosine are respectively in a ratio of 15:55:1.5 by weight.

**Patentansprüche**

1. Verwendung von Roßkastanienextrakt, Oxyethophyllin und Adenosin für die Herstellung einer Zusammensetzung zum Erhöhen des hochdichten Lipoproteincholesterins im Blut.
2. Verwendung nach Anspruch 1, wobei der Roßkastanienextrakt, das Oxyethophyllin und das Adenosin jeweils in einem Gewichtsverhältnis von 15:55:1,5 vorliegen.

**Revendications**

1. Utilisation de l'extrait de marron d'Inde, de l'oxyéthophylline et de l'adénosine pour la manufacture d'une composition pour augmenter de lipoprotéine cholesterol de haute densité du sang.
2. Utilisation suivant la revendication 1, sous laquelle l'extrait de marron d'Inde, l'oxyéthophylline et l'adénosine sont en proportion en poids de 15:55:1.5.